# EUROPEAN PATENT APPLICATION

(11) **EP 4 173 714 A1**
(43) Date of publication of application: **03.05.2023**
(21) Application number: 21306501.4
(22) Date of filing: 27.10.2021
(51) Int. Cl.: B01L 3/00, A61B 10/00, G01N 1/02

(54) **DEVICE FOR COLLECTING BIOLOGICAL SAMPLES**

(71) Applicant: BIC Violex Single Member S.A., 14569 Anoixi (GR); Société BIC, 92110 Clichy (FR)
(72) Inventor: Katsikas, Georgios, 145 69 Anoixi (GR); Saltas, Efthymios, 145 69 Anoixi (GR); Michenaud, Etienne, 92110 Clichy (FR)
(74) Representative: Peterreins Schley

(57) **Abstract**

The present disclosure relates to a device for collecting one or more biological samples comprising a housing and a collector element, wherein the collector element is designed to collect one or more biological samples. The device comprises a cap element which can be mounted on the housing, wherein the cap element comprises a reagent or an element comprising a reagent.

## Description

### Background of the present disclosure

The present disclosure relates to device for collecting one or more biological samples, which may be subsequently used for molecular biology processing techniques such as nucleic acid amplification and/or detection.

Polymerase Chain Reaction (PCR) is a known method of amplifying nucleic acids, employed to test for the presence of specific nucleic acids (DNA or RNA) in a biological sample. It is used among other purposes as a diagnostic method to identify markers for pathogens or diseases. Other methods of amplifying nucleic acid samples include isothermal amplification methods such as Recombinase Polymerase Amplification (RPA) and Loop-Mediated Isothermal Amplification (LAMP).

Devices for collecting one or more biological samples - in particular for collecting saliva from the mouth of the consumer - are known from WO 2020/191232 A1, WO 2020/106345 A1, WO 2020/238035 A1 and EP 3 028 030 A1. Those devices comprise a wick as collector element. However, the wick is very soft and is difficult to maintain and fasten into the housing of the device.

The object of the present disclosure is to provide a device for collecting one or more biological samples, wherein the one or more biological samples can be tested and/or analyzed more easily.

### Summary of the present disclosure

The present disclosure is directed to a device for collecting one or more biological samples as defined in independent claim 1. The dependent claims depict embodiments of the present disclosure.

According to the present disclosure, a device for collecting one or more biological samples is provided comprising a housing and a collector element, wherein the collector element is designed to collect one or more biological samples. The device comprises a cap element which can be mounted on the housing, wherein the cap element comprises a reagent or an element comprising a reagent. With that, it is possible to test/analyze the one or more biological samples on site, if desired.

As will be explained below in detail, the device may comprise a container for storing liquid. The collector element may be designed to allow the passage of the liquid so that at least part of the liquid can pass through a channel and through the collector element to wash out the one or more biological samples out of the collector element at least partially for testing/analyzing purposes.

For testing/analyzing the one or more biological samples, the reagent or the element comprising a reagent may be contained in the cap which can be mounted on the housing for covering the collector element. When the mixture of liquid from the container and of the at least one biological sample reaches a reaction chamber of the cap, the reagent is released to the mixture for testing/analyzing the one or more samples on site.

It should be noted that the housing may consist of several separate housing parts. The collector element may generally have an elongated shape extending from the first end to the second end of the collector element. The cross-sectional shape of the collector element (in a plane which is perpendicular to the axis of the collector element) may at least partially be circular, oval or rectangular (in examples with rounded corners). The section of the collector element at its first end may have a conical section. In addition or alternatively, the first end of the collector element may be pointed.

The purpose of the collector element is to collect and/or absorb one or more biological samples. In case of dry biological samples, the biological samples may stick on the outer surface of the first end of the collector element. In case of biological samples which are contained in a liquid, the biological samples may be absorbed within the collector element. Therefore, the collector element should allow the passage of liquid at least partially in order to take up absorb a greater amount of the biological sample(s).

Suitable materials for the collector element include cotton-based materials, or a plastic based matrix such as PE (polyethylene), PP (polypropylene) or PVDF (polyvinylidene fluoride) or a combination thereof, which can be manufactured in a range of densities to retain biological samples of different viscosities. Most specifically, the collector element may be made of a combination of PE and PP, wherein the PP may be the core portion and the PE may be the outer coating, due to its particular properties on absorption of liquid via its capillary structure, and adsorption which is minimal with regards to nucleic acids. The collector element may comprise fibers (in examples, pressed fibers) made of one or more of these materials. The material of the collector element may be porous or may have an internal channel structure, and may have hydrophilic properties in order to absorb a large amount of liquid biological samples more quickly. The porosity may be at least 50%, more specifically at least 70%, most specifically at least 80%.

The collector element may extend between a first end and a second end, the first end of the collector element protruding out of the housing for collecting the one or more biological samples, the second end of the collector element being arranged within the housing. The collector element may allow the passage of the liquid from the container, in particular a buffer solution and/or a reagent solution, through the collector element in order to drain the biological sample(s) out of the collector element. In particular, the collector element may allow the passage of liquid from its second end which is arranged in the housing, to its first end which protrudes out of the housing, or from the outer surface near the second end to the first end.

An element for indicating and/or measuring a reaction between the reagent and the at least one biological sample may be arranged within or on the cap element, in particular an element for indicating the pH value. The liquid from the container drains the biological sample(s) into the cap element, and the reagent is then released to the liquid containing the biological sample(s). This may cause a reaction which may result in a certain pH value which can be indicated by the element for indicating the pH value, in particular a pH strip indicating a certain color dependent on the specific pH value. In other examples, the reaction may cause a fluorescence effect so that a certain reaction is indicated by the emitted fluorescence light. A window may be provided in the cap element so that the user can see the effect of the reaction (e.g. a certain color of the pH strip or the emitted fluorescence light). In other examples, the cap element may be made of or comprise a transparent material.

The cap element may comprise a reaction chamber, and wherein the reagent or the element comprising a reagent and/or the element for indicating and/or measuring a reaction is arranged within the reaction chamber. In addition, a holder may be arranged within the reaction chamber. The holder may be designed to premount the reagent or the element comprising a reagent and/or the element for indicating and/or measuring a reaction on or within the holder, before the holder is mounted within the reaction chamber. This ensures that one or both elements are securely positioned within the reaction chamber to ensure a proper test result. The holder may hold the reagent or the element comprising a reagent and/or the element for indicating and/or measuring a reaction by shape-fit, snap-fit and/or press-fit features. The holder itself may also be held within the reaction chamber by shape-fit, snap-fit and/or press-fit features.

The cap element may have an opening so that liquid from the container which has passed through the collector element can reach the reaction chamber within the cap. This opening may at least partially comprise a converging shape for funneling the liquid into the reaction chamber. Alternatively or in addition, the holder may have an end portion facing the collector element which at least partially comprises a converging shape for funneling a liquid into the reaction chamber.

The cap element may be held on the housing by shape-fit, snap-fit and/or press-fit features to securely hold the cap element in position during use, in particular when the liquid drains the sample(s) into the reaction chamber. In addition, the cap element may comprise ribs and/or protrusions for engaging at least part of the front portion of the housing for holding the cap element in a predefined position.In examples, the predefined position may be in an axially aligned position with respect to the longitudinal axis of the housing.

As already mentioned, the device may comprise a container for storing liquid and a channel for guiding the liquid to the collector element. The container may initially be in a closed state for storing the liquid and may further comprise a release mechanism for opening the container or a passage from the container to the channel so that liquid stored in the container can enter the channel from the container and reach the collector element. For example, the container may have an opening which is closed by a membrane, wherein the release mechanism comprises a cutting or penetrating member for opening the container by a cutting or penetrating actuation. This can - for example - be achieved by moving the container from a first position into (or further into) the housing to a second position, wherein the cutting/penetrating member may be fixedly arranged within the housing. In this case, the membrane is closed in the first position of the container, and wherein the membrane is cut open or penetrated by the cutting or penetrating member in the second position of the container.

The liquid can thus be released when needed, i.e. after the one or more samples have been collected, and when a user wants to analyze/test the sample(s). In this case, liquid stored in the container can reach the collector element through the channel when the release mechanism is open, wherein at least part of the liquid can pass through the collector element and into the reaction chamber, for example by gravity acting on the liquid when the container is in a position vertically above the collector element. In other examples, the container may be compressible to force the liquid through the collector element.

In embodiments, the housing and the container may also have a mechanism to retain the container in its first position such that the initial actuation force required to move the container into the housing is higher than the subsequent actuation force needed to cut open or penetrate the membrane. This feature ensures that the first position is maintained as a preset position. As an example, the retaining mechanism may comprise at least two internal protrusions on the inner side of the housing forming a circumferential groove between the protrusions, and a circumferential protrusion on the outer side of the container, wherein the circumferential protrusion is held within the circumferential groove of the housing. Therefore, when moving the container from its first position to its second position, the protrusion on side of the container needs to be pushed or moved over the circumferential protrusion on the housing which is closer to the cutting/penetrating member. This actuation requires an increased initial force which also avoids an undesired release of the liquid from the container.

In addition or alternatively, the housing may comprise a locking mechanism for maintaining the container in its first position, and which can be unlocked so that the container can be moved into its second position. This locking mechanism requires an unlocking actuation by a user which is different compared to a pushing or moving actuation on the container itself. For example, the locking mechanism may comprise at least one lever which can be operated by a user to disengage a protrusion on the container (which protrusion may be identical to the previously mentioned protrusion on the container). As another example, the locking mechanism may comprise a bayonet connection such that the container must be rotated first to unlock the axial position of the container so that the container can subsequently be moved in axial direction into the housing.

The device of the present disclosure may be in a state in which the container does not yet contain the liquid. In other examples, the container may already comprise a liquid, in particular a buffer solution and/or a reagent solution, i.e. the device may be in prefilled state.

According to this disclosure, various amplification methods may be used (i.e. the device of the present disclosure may be designed for carrying out one of these methods), for example a recombinase polymerase amplification (RPA) which is a low temperature DNA and/or RNA amplification technique, or a ligase chain reaction (LCR).

The liquid stored in the container may be a buffer liquid and may contain purified water, milli-q water and buffer (e.g. TRIS-Cl / TE buffer), for example a Tris-HCl buffer. When LCR method is applied, the buffer liquid also contains PEG as crowding agent.

The reagents located in the cap may include a group of materials, such as primers, probes additionally to the enzymes (ligase or polymerase) and nucleotides. Generally, the reagents located in the cap are dependent on the used amplification method. A suitable reagent composition for RPA may contain enzymes (in particular enzymes for polymerase and/or enzymes for reverse transcriptase), proteins, primers, probes, oligonucleotides and reaction components necessary for the RPA reaction including reaction buffer, polyethylene glycol, pyrophosphatase, a regeneration system such as kinase/phosphocreatine system, or a mixture thereof. A suitable reagent composition for LCR may contain enzymes for DNA ligase and/or DNA probes.

Possible materials for the collector element have already been described. The collector element may additionally be treated. For example, reagents or active agents may be covalently bound to the collector element by chemical linkages through functionalisation (e.g. -OH groups), or they may be dried into the material of the collector element and become active on hydration with the sample. Alternatively or in addition, the collector element may be treated to provide means of lysing virus, in particular a treatment to form a negatively charged surface (so as to retain the positively charged ions of the sample) or a treatment with anionic detergent. Possible active agents that render the collector element functionalized may lyse bacteria or viruses. These active agents may be quaternary ammonium compounds (QAC) and/or biocidal agents. Examples of these active agents are: Citral, Eucalyptus oil, Tea tree oil, Chlorhexidine, triterpenoid saponin, Polyphylla saponin I, Povidone-iodine, Cetyl pyridinium chloride (CPC), Benzalkonium chloride (BAC), Dequoalinium chloride.

### Short description of the figures

- Figure 1: shows a device for collecting one or more biological samples according to an embodiment of the present disclosure;
- Figure 2: shows a cross-section through a device for collecting one or more biological samples according to an embodiment of the present disclosure;
- Figure 3: shows a cap element according to an embodiment the present disclosure including the parts which may be arranged within the cap element in a disassembled state;
- Figures 4a to 4c: show a holder according to an embodiment of the present disclosure and an element for indicating a reaction;
- Figure 5: shows a holder according to an embodiment of the present disclosure together with an element for indicating a reaction and an element containing a reagent in a premounted state;
- Figure 6: shows a cap element according to an embodiment of the present disclosure and a holder in a state;
- Figures 7a and 7b: show a holder according to an embodiment of the present disclosure in its mounted state within a cap element;
- Figures 8a to 8c: show a cap element and a holder according to another embodiment of the present disclosure together with an element for indicating a reaction and an element containing a reagent;
- Figures 9, 10a and 10b: show a device for collecting one or more biological samples according to another embodiment of the present disclosure;
- Figure 11: shows a cap element according to another embodiment of the present disclosure together with a reagent and/or an element for indicating a reaction.

### Detailed description of the present disclosure

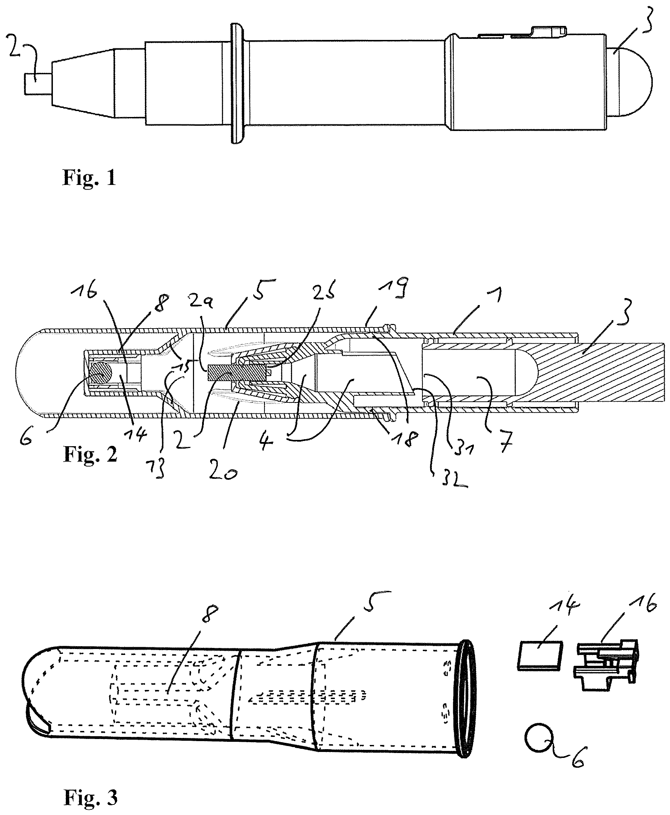

Figures 1 shows a device for collecting one or more biological samples according to an embodiment of the present disclosure. Figure 2 shows a cross-section through a device for collecting one or more biological samples according to another embodiment of the present disclosure which is a variation of the embodiment of Figure 1.

The device of the present disclosure comprises a housing 1 and a collector element 2, wherein the collector element is designed to collect one or more biological samples. The device further comprises a cap element 5 (not shown in Figure 1) which can be mounted on the housing, wherein the cap element 5 comprises a reagent 6 or an element comprising a reagent. With that, it is possible to test/analyze the one or more biological samples on site, if desired.

As will be explained below in detail, the device may comprise a container 3 for storing liquid 7. The collector element 2 may be designed to allow the passage of the liquid 7 so that at least part of the liquid can pass through a channel 4 and through the collector element 2 to wash out the one or more biological samples out of the collector element at least partially for testing/analyzing purposes. For testing/analyzing the one or more biological samples, the reagent 6 or the element comprising a reagent is contained a reaction chamber 8 within the cap element 5. When the mixture of liquid from the container and of the at least one biological sample reaches a reaction chamber of the cap, the reagent is released to the mixture for testing/analyzing the one or more samples on site.

The purpose of the collector element 2 is to collect and/or absorb one or more biological samples. In case of dry biological samples, the biological samples may stick on the outer surface of the first end 2a of the collector element. In case of biological samples which are contained in a liquid, the biological samples may be absorbed within the collector element. Therefore, the collector element should allow the passage of liquid at least partially in order to take up absorb a greater amount of the biological sample(s). The material of the collector element may be porous or may have an internal channel structure, and may have hydrophilic properties in order to absorb a large amount of liquid biological samples more quickly.

Suitable materials for the collector element 2 include cotton-based materials, or a plastic based matrix such as PE (polyethylene), PP (polypropylene) or PVDF (polyvinylidene fluoride) or a combination thereof, which can be manufactured in a range of densities to retain biological samples of different viscosities. Most specifically, the collector element is made of a combination of PE and PP, wherein the PP is the core portion and the PE is the outer coating, due to its particular properties on absorption of liquid via its capillary structure, and adsorption which is minimal with regards to nucleic acids. The collector element may comprise fibers (in examples, pressed fibers) made of one or more of these materials. The material of the collector element may be porous or may have an internal channel structure, and may have hydrophilic properties in order to absorb a large amount of liquid biological samples more quickly. The porosity may be at least 50%, more specifically at least 70%, most specifically at least 80%. The density of the collector element may range from 0.1 to 0.25 g/cm³.

The collector element 2 may have two ends, the first end 2a of the collector element protruding out of the housing for collecting the one or more biological samples, the second end 2a of the collector element being arranged within the housing. The collector element may allow the passage of the liquid from the container 3, in particular a buffer solution and/or a reagent solution, through the collector element in order to drain the biological sample(s) out of the collector element and into the reaction chamber 8. In particular, the collector element may allow the passage of liquid from its second end which is arranged in the housing, to its first end which protrudes out of the housing, or from the outer surface near the second end to the first end.

Figure 3 shows a cap element according to an embodiment the present disclosure including the parts which may be arranged within the cap element in a disassembled state, in particular the reagent 6 (or the element comprising a reagent). The reagent 6 (or the element comprising a reagent) may have any shape; in the shown embodiment, it has the shape of a sphere.

In this embodiment, an element 14 for indicating and/or measuring a reaction between the reagent and the at least one biological sample is also arranged within or on the cap element, in particular an element 14 or a strip for indicating the pH value. The liquid 7 from the container 3 drains the biological sample(s) into the cap element 5, and the reagent 6 is then released to the liquid containing the biological sample(s). This may cause a reaction which may result in a certain pH value which can be indicated by the element 14 for indicating the pH value, in particular a pH strip indicating a certain color dependent on the specific pH value.

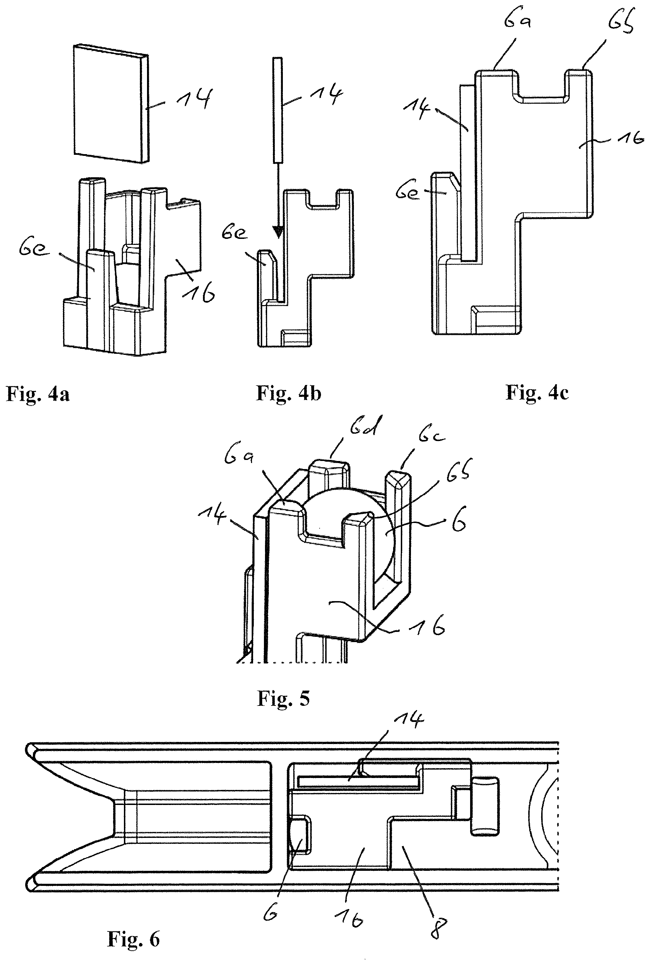

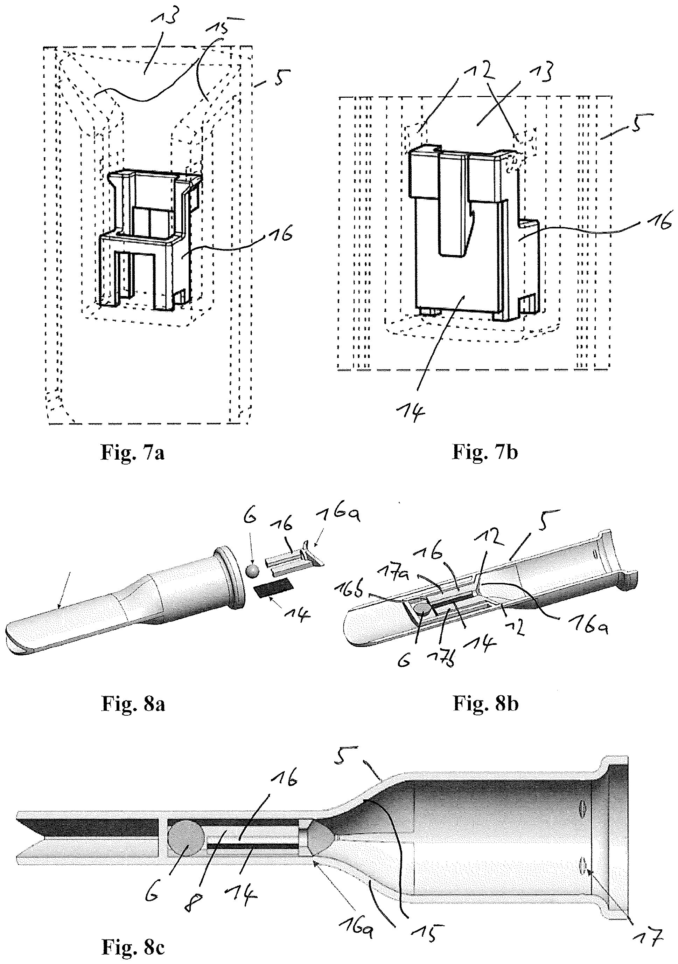

As shown in Figures 6 and 7a, the cap element 5 may comprise a reaction chamber 8, wherein the reagent 6 (or the element comprising a reagent) and/or the element 14 for indicating and/or measuring a reaction are arranged within the reaction chamber 8.

In addition, a holder 16 as shown in Figures 3 to 5 may be arranged within the reaction chamber (see also Figure 6). The holder 16 may be designed to premount the reagent 6 (or the element comprising a reagent) and/or the element 14 for indicating and/or measuring a reaction on or within the holder 6, before the holder is mounted within the reaction chamber. Figures 4a to 4c show how the element 14 (for example a pH strip or an element comprising a reagent for causing a fluorescence effect when coming into contact with the biological sample or the mixture of the reagent and the biological sample) is mounted on the holder 16. Figure 5 shows the premounted state in which both, the element 14 and the reagent 6 are mounted on the holder 16. The holder 16 ensures that the element 14 and the reagent 6 are securely positioned within the reaction chamber 8 to ensure a proper test result.

The holder 16 may hold the reagent 6 (or the element comprising a reagent) and/or the element for indicating and/or measuring a reaction by shape-fit, snap-fit and/or press-fit features. In the embodiment shown in Figures 4a to 5, the reagent 6 is held by four posts 6a to 6d which hold the reagent 6 (or the element comprising a reagent) by a press-fit, i.e. the inner free diameter of the posts 6a to 6d is slightly smaller than the diameter the spherical shape of the reagent 6. Of course, the posts 6a to 6d may also have recesses (in particular concave recesses) for achieving a shape-fit for holding the reagent 6. In the shown embodiment, the element 14 is also held by a post 6e by press-fit, i.e. the distance of the gap formed between the posts 6a/6b and the post 6e is slightly smaller than the width of the pH strip.

The holder itself may also be held within the reaction chamber by shape-fit, snap-fit and/or press-fit features. For example, in the embodiment shown in Figure 6, the cap element 5 may have one or more internal protrusions 12 which can be slightly deformed when the holder 16 is inserted into the reaction chamber 8, and which serve to correctly position the holder 16 within the reaction chamber 8 (as shown in Figures 6, 7a and 7b).

The cap element 5 may have an opening 13 (see Figures 2, 7a and 7b) so that liquid from the container 3 which has passed through the collector element 2 can reach the reaction chamber 8 within the cap element 5. This opening may at least partially comprise a converging shape 15 for funneling the liquid into the reaction chamber (see Figures 2 and 7a). Alternatively or in addition, as shown in Figures 8a to 8c, the holder 16 may have an end portion 16a facing the collector element which at least partially comprises a converging shape for funneling the liquid into the reaction chamber 8.

Figures 8a to 8c shows a cap element 5 and a holder 16 according to another embodiment of the present disclosure together with an element 14 for indicating a reaction and a reagent 6 (or an element containing a reagent). In this embodiment, only the element 14 for indicating and/or measuring a reaction is premounted on the holder 16 by two posts 17a, 17b which have two internal grooves extending in longitudinal direction for inserting the element 14. The reagent 6 (or an element containing the reagent) is held within the reaction chamber 8 by the distal end 16b of the holder 16. Therefore, the reagent 6 is placed into the reaction chamber 8 first. Thereafter, the holder 16 (together with the premounted element 14) is inserted into the reaction chamber 8 for holding the reagent 6 in place. Again, the holder 16 may be mounted within the cap element 5 by shape-fit, snap-fit and/or press-fit features. In the shown embodiment, one or more protrusions 12 are provided on the inner side of the cap element for providing a snap-fit of the holder 16 within the reaction chamber 8.

In the embodiments shown in Figures 8a to 8c, the cap element has an opening 13 which has at least partially comprise a converging shape 15 for funneling the liquid into the reaction chamber. In addition, the holder 16 has an end portion 16a facing the collector element 2 which at least partially comprises a converging shape for funneling the liquid into the reaction chamber 8.

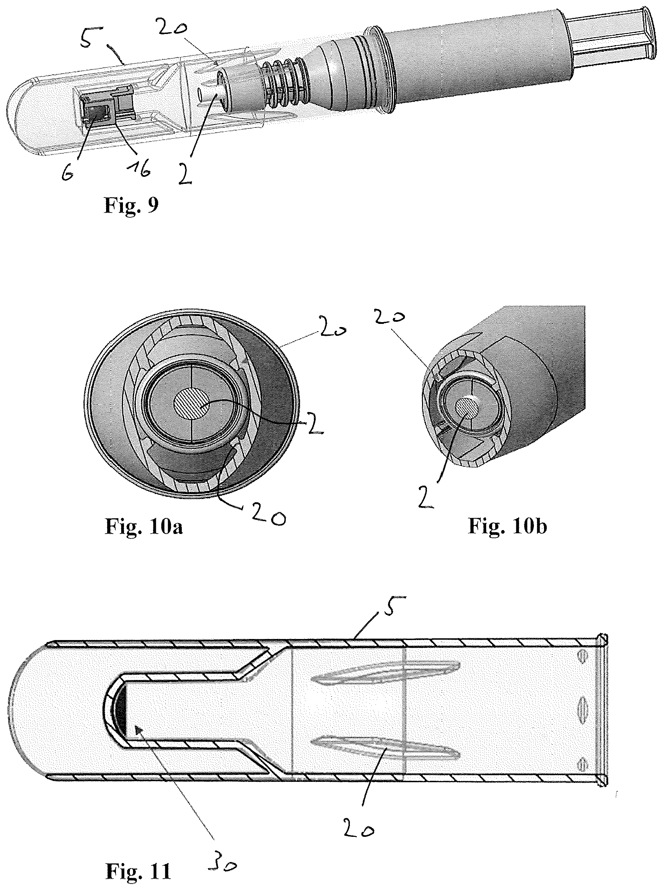

Figure 11 shows a cap element according to another embodiment of the present disclosure together with a substance 30 which may comprise a reagent and/or a substance for indicating a reaction. This embodiment does not require a holder as discussed above, whereas it should be understood that a holder may be used. Instead, the substance 30 is directly held at the bottom or on an internal surface of the reaction chamber 8. For example, the substance 30 may be applied in a dissolved state to the reaction chamber 8, and then dried so that the substance 30 adheres to the bottom or on an internal surface of the reaction chamber 8.

The biological sample may react with the reagent and/or the substance for indicating a reaction. For example, the reaction may cause a fluorescence effect (either auto-fluorescence or fluorescence caused by applying an excitation light) so that the user can directly see the result of the reaction via the fluorescence effect. For this purpose, a window may be provided in the cap element, or the cap element may comprise or be made of a transparent material.

In all embodiments, the cap element may be held on the housing by shape-fit, snap-fit and/or press-fit features to securely hold the cap element in position during use, in particular when the liquid drains the sample(s) into the reaction chamber. For example, Figure 8c shows protrusions 17 on the inner side of the cap element 5 which are designed to engage a circumferential groove on the housing.

In other examples, the cap element 5 may be fitted on the housing by a shape-fit as shown in Figure 2, for example by providing a circumferential protrusion 18 on the housing which abuts the proximal end 19 of the cap element 5. In addition, as shown in Figures 9 to 10b, the cap element may comprise ribs 20 and/or protrusions for engaging at least part of the front portion of the housing for holding the cap element in a predefined position, which may be in an axially aligned position with respect to the longitudinal axis of the housing.

As already mentioned, the device may comprise a container for storing liquid and a channel for guiding the liquid to the collector element. The container may initially be in a closed state for storing the liquid and may further comprise a release mechanism for opening the container or a passage from the container to the channel so that liquid stored in the container can enter the channel from the container and reach the collector element. For example, the container may have an opening which is closed by a membrane 31, wherein the release mechanism comprises a cutting or penetrating member 32 for opening the container by a cutting or penetrating actuation (see Fig. 2). This can - for example - be achieved by moving the container from a first position into (or further into) the housing to a second position, wherein the cutting/penetrating member may be fixedly arranged within the housing. In this case, the membrane is closed in the first position of the container, and wherein the membrane is cut open or penetrated by the cutting or penetrating member in the second position of the container.

The liquid can thus be released when needed, i.e. after the one or more samples have been collected, and when a user wants to analyze/test the sample(s). In this case, liquid stored in the container can reach the collector element through the channel when the release mechanism is open, wherein at least part of the liquid can pass through the collector element and into the reaction chamber, for example by gravity acting on the liquid when the container is in a position vertically above the collector element. In other examples, the container may be compressible to force the liquid through the collector element.

The housing and the container may also have a mechanism to retain the container in its first position such that the initial actuation force required to move the container into the housing is higher than the subsequent actuation force needed to cut open or penetrate the membrane. This feature ensures that the first position is maintained as a preset position. As an example, the retaining mechanism may comprise two internal protrusions on the inner side of the housing forming a circumferential groove between the protrusions, and a circumferential protrusion on the outer side of the container, wherein the circumferential protrusion is held within the circumferential groove of the housing. Therefore, when moving the container from its first position to its second position, the protrusion on side of the container needs to be pushed or moved over the circumferential protrusion on the housing which is closer to the cutting/penetrating member. This actuation requires an increased initial force which also avoids an undesired release of the liquid from the container.

In addition or alternatively, the housing may comprise a locking mechanism for maintaining the container in its first position, and which can be unlocked so that the container can be moved into its second position. This locking mechanism requires an unlocking actuation by a user which is different compared to a pushing or moving actuation on the container itself. For example, the locking mechanism may comprise at least one lever which can be operated by a user to disengage a protrusion on the container (which protrusion may be identical to the previously mentioned protrusion on the container). As another example, the locking mechanism may comprise a bayonet connection such that the container must be rotated first to unlock the axial position of the container so that the container can subsequently be moved in axial direction into the housing.

The device of the present disclosure may be in a state in which the container does not yet contain the liquid. In other examples, the container may already comprise a liquid, in particular a buffer solution and/or a reagent solution, i.e. the device may be in prefilled state.

According to this disclosure, various amplification methods may be used (i.e. the device of the present disclosure may be designed for carrying out one of these methods), for example a recombinase polymerase amplification (RPA) which is a low temperature DNA and/or RNA amplification technique, or a ligase chain reaction (LCR).

The liquid stored in the container may be a buffer liquid and may contain purified water, milli-q water and buffer (e.g. TRIS-Cl / TE buffer), for example a Tris-HCl buffer. When LCR method is applied, the buffer liquid also contains PEG as crowding agent.

The reagents located in the cap may include a group of materials, such as primers, probes additionally to the enzymes (ligase or polymerase) and nucleotides. Generally, the reagents located in the cap are dependent on the used amplification method. A suitable reagent composition for RPA may contain enzymes (in particular enzymes for polymerase and/or enzymes for reverse transcriptase), proteins, primers, probes, oligonucleotides and reaction components necessary for the RPA reaction including reaction buffer, polyethylene glycol, pyrophosphatase, a regeneration system such as kinase/phosphocreatine system, or a mixture thereof. A suitable reagent composition for LCR may contain enzymes for DNA ligase and/or DNA probes.

Possible materials for the collector element have already been described. The collector element may additionally be treated. For example, reagents or active agents may be covalently bound to the collector element by chemical linkages through functionalisation (e.g. -OH groups), or they may be dried into the material of the collector element and become active on hydration with the sample. Alternatively or in addition, the collector element may be treated to provide means of lysing virus, in particular a treatment to form a negatively charged surface (so as to retain the positively charged ions of the sample) or a treatment with anionic detergent. Possible active agents that render the collector element functionalized may lyse bacteria or viruses. These active agents may be quaternary ammonium compounds (QAC) and/or biocidal agents. Examples of these active agents are: Citral, Eucalyptus oil, Tea tree oil, Chlorhexidine, triterpenoid saponin, Polyphylla saponin I, Povidone-iodine, Cetyl pyridinium chloride (CPC), Benzalkonium chloride (BAC), Dequoalinium chloride.

Although the device of the present disclosure is defined in the following claims, it should be understood that the present disclosure can alternatively and/or in addition be defined according to the following embodiments:
1. A device for collecting one or more biological samples comprising a housing and a collector element,
   wherein the collector element is designed to collect one or more biological samples,
   wherein the device further comprises a cap element which can be mounted on the housing, wherein the cap element comprises a reagent or an element comprising a reagent.
2. A device according to embodiment 1, wherein an element for indicating and/or measuring a reaction between the reagent and the at least one biological sample is arranged within or on the cap element.
3. A device according to embodiment 2, wherein the element for indicating a reaction indicates the pH value of a solution comprising the reagent and the at least one biological sample or causes a fluorescence effect of a solution comprising the reagent and the at least one biological sample.
4. A device according to one of the preceding embodiments, wherein the cap element comprises a reaction chamber, and wherein the reagent or the element comprising a reagent and/or the element for indicating and/or measuring a reaction is arranged within the reaction chamber.
5. A device according to one of the preceding embodiments, wherein a holder is arranged within the reaction chamber, wherein the holder is designed to premount the reagent or the element comprising a reagent and/or the element for indicating and/or measuring a reaction on or within the holder, before the holder is mounted within the reaction chamber.
6. A device according to one of the preceding embodiments, wherein the holder holds the reagent or the element comprising a reagent and/or the element for indicating and/or measuring a reaction by shape-fit, snap-fit and/or press-fit features.
7. A device according to one of the preceding embodiments, wherein the holder is held within the reaction chamber by shape-fit, snap-fit and/or press-fit features.
8. A device according to one of the preceding embodiments, wherein the cap element has an opening which at least partially comprises a converging shape for funneling a liquid into the reaction chamber.
9. A device according to one of the preceding embodiments, wherein the holder has an end portion which at least partially comprises a converging shape for funneling a liquid into the reaction chamber.
10. A device according to one of the preceding embodiments, wherein the cap element comprises ribs and/or protrusions for engaging at least part of the front portion of the housing for holding the cap element in a predefined position.
11. A device according to one of the preceding embodiments, wherein the cap element is held on the housing by shape-fit, snap-fit and/or press-fit features.
12. A device according to one of the preceding embodiments, wherein the device comprises a container for storing liquid and a channel for guiding the liquid to the collector element.
13. Device according to one of the preceding embodiments, wherein the collector element has two ends, the first end of the collector element protruding out of the housing for collecting the one or more biological samples, the second end of the collector element being arranged within the housing.
14. Device according to one of the preceding embodiments, wherein the collector element is designed to allow the passage of the liquid so that at least part of the liquid can pass through the channel and through the collector element to wash out the one or more biological samples out of the collector element at least partially for testing/analysis purposes.
15. Device according to one of the preceding embodiments, wherein the container is initially in a closed state for storing the liquid and further comprises a release mechanism for opening the container or a passage from the container to the channel so that liquid stored in the container can enter the channel from the container and reach the collector element.
16. Device according to one of the preceding embodiments, wherein the container has an opening which is closed by a membrane, and wherein the release mechanism comprises a cutting or penetrating member for opening the container by a cutting or penetrating actuation.
17. Device according to one of the preceding embodiments, wherein the container and the housing are designed such that the container can be moved from a first position into the housing to a second position.
18. Device according to one of the preceding embodiments, wherein the membrane is closed in the first position of the container, and wherein the membrane is cut open or penetrated by the cutting or penetrating member in the second position of the container.
19. Device according to one of the preceding embodiments, wherein liquid stored in the container can reach the collector element through the channel when the release mechanism is open, wherein at least part of the liquid can pass through the collector element and into the reaction chamber by means of gravity acting on the liquid when the container is in a position vertically above the collector element.

## Claims

1. A device for collecting one or more biological samples comprising a housing and a collector element,
wherein the collector element is designed to collect one or more biological samples, and wherein the device comprises a cap element which can be mounted on the housing, wherein the cap element comprises a reagent or an element comprising a reagent.

2. A device according to claim 1, wherein an element for indicating and/or measuring a reaction between the reagent and the at least one biological sample is arranged within or on the cap element.

3. A device according to claim 2, wherein the element for indicating a reaction indicates the pH value of a solution comprising the reagent and the at least one biological sample or causes a fluorescence effect of a solution comprising the reagent and the at least one biological sample.

4. A device according to one of the preceding claims, wherein the cap element comprises a reaction chamber, and wherein the reagent or the element comprising a reagent and/or the element for indicating and/or measuring a reaction is arranged within the reaction chamber.

5. A device according to one of the preceding claims, wherein a holder is arranged within the reaction chamber, wherein the holder is designed to premount the reagent or the element comprising a reagent and/or the element for indicating and/or measuring a reaction on or within the holder, before the holder is mounted within the reaction chamber.

6. A device according to claim 5, wherein the holder holds the reagent or the element comprising a reagent and/or the element for indicating and/or measuring a reaction by shape-fit, snap-fit and/or press-fit features.

7. A device according to one of the preceding claims, wherein the reagent has a spherical shape.

8. A device according to one of the preceding claims, wherein the cap element has an opening which at least partially comprises a converging shape for funneling a liquid into the reaction chamber.

9. A device according to one of the preceding claims, wherein the holder has an end portion which at least partially comprises a converging shape for funneling a liquid into the reaction chamber.

10. A device according to one of the preceding claims, wherein the cap element comprises ribs and/or protrusions for engaging at least part of a front portion of the housing for holding the cap element in a predefined position.

11. A device according to one of the preceding claims, wherein the cap element is held on the housing by shape-fit, snap-fit and/or press-fit features.

12. A device according to one of the preceding claims, wherein the device comprises a container for storing liquid and a channel for guiding the liquid to the collector element.

13. Device according to one of the preceding claims, wherein the collector element is designed to allow the passage of the liquid so that at least part of the liquid can pass through the channel and through the collector element to wash out the one or more biological samples out of the collector element at least partially for testing/analysis purposes.

14. Device according to one of the preceding claims, wherein the container is initially in a closed state for storing the liquid and further comprises a release mechanism for opening the container or a passage from the container to the channel so that liquid stored in the container can enter the channel from the container and reach the collector element.

15. Device according to one of the preceding claims, wherein liquid stored in the container can reach the collector element through the channel when the release mechanism is open, wherein at least part of the liquid can pass through the collector element and into the reaction chamber by gravity acting on the liquid when the container is in a position vertically above the collector element.
